# EUROPÄISCHE PATENTSCHRIFT

(11) **EP 2 004 080 B1**
(45) Veröffentlichungstag und Bekanntmachung des Hinweises auf die Patenterteilung: **16.07.2014**
(21) Anmeldenummer: 07723438.3
(22) Anmeldetag: 20.03.2007
(51) Int. Cl.: A61B 18/12

(54) **VERBINDUNGSKABEL**
CONNECTION CABLE
CABLE DE CONNEXION

(30) Priorität: 05.04.2006 DE 102006015972; 15.05.2006 DE 102006022606
(43) Veröffentlichungstag der Anmeldung: 24.12.2008
(73) Patentinhaber: ERBE Elektromedizin GmbH, 72072 Tübingen (DE)
(72) Erfinder: BELLER, Jürgen, 72810 Gomaringen (DE); HAGG, Martin, 72827 Wannweil (DE); SCHNITZLER, Uwe, 72074 Tübingen (DE)
(74) Vertreter: Bohnenberger, Johannes
(86) Internationale Anmeldenummer: PCT/EP2007/002476
(87) Internationale Veröffentlichungsnummer: WO 2007/115655

(56) Entgegenhaltungen:
- WO-A-02/41798

## Beschreibung

Die Erfindung betrifft ein Verbindungskabel zum Verbinden eines HF-chirurgischen Instrumentes mit einem HF-chirurgischen Gerät nach dem Oberbegriff des Patentanspruches 1, wie sehen aus WO 0 241 798 bekannt.

Es gibt verschiedene hochfrequenzchirurgische Geräte. Ein HF-chirurgisches Gerät besteht beispielsweise aus mindestens einem Hochfrequenz-Generator zur Erzeugung hochfrequenter elektrischer Spannungen bzw. Ströme zum monopolaren, bipolaren oder quasi bipolaren Schneiden und/oder Koagulieren biologischer Gewebe. Weiterhin sind Einrichtungen zur Einstellung, Überwachung, Regelung, Begrenzung und/oder Modulation der zum Schneiden und/oder Koagulieren erforderlichen HF-Spannungen, HF-Ströme, elektrischen Lichtbogen zwischen aktiver Elektrode und biologischem Gewebe und/oder der Leistung vorhanden. HF-chirurgische Geräte können außerdem mit verschiedenen Betriebsmodi zum Schneiden und/oder Koagulieren ausgestattet sein, wie beispielsweise Soft-Koagulation, forcierte Koagulation, Spray-Koagulation, kontinuierlich im Schnitt oder fraktioniert im Schnitt und automatische Anschnittsteuerung. HF-chirurgische Geräte können außerdem mit Einrichtungen zur manuellen und/oder automatischen Aktivierung und/oder automatischen Deaktivierung, automatischen Begrenzung der Aktivierungsdauer, Einrichtungen zur automatischen Überwachung verschiedener Sicherheitskriterien etc. ausgestattet sein.

HF-chirurgische Instrumente wiederum sind beispielsweise monofunktionale, bifunktionale oder multifunktionale Instrumente zum monopolaren, bipolaren oder quasi bipolaren Schneiden und/oder Koagulieren biologischer Gewebe, die ebenso wie die HF-chirurgischen Geräte von verschiedenen Herstellern in den verschiedensten Formen angeboten werden.

Dadurch, dass das Angebot verschiedener Instrumente und Geräte für die Hochfrequenzchirurgie seit der Entwicklung der minimal-invasiven Chirurgie in fast allen chirurgischen Fachbereichen immer größer geworden ist, steigen auch die Anforderungen an das Personal bezüglich der richtigen Einstellung des an diesen Instrumenten angeschlossenen HF-chirurgischen Gerätes. Ein in Bezug auf das angeschlossene HF-chirurgische Instrument falsch eingestellter Betriebsmodus oder eine zu hoch eingestellte HF-Spannung, HF-Leistung oder Intensität des HF-Stroms kann ein hierfür nicht geeignetes Instrument zerstören oder dem Patienten Schaden zufügen.

Weiterhin wird bei einem Wechsel des vom Chirurgen verwendeten HF-chirurgischen Instrumentes während einer Operation in der Regel eine Änderung der Einstellung des mit verwendeten HF-chirurgischen Gerätes notwendig, was die Aufmerksamkeit des Operationsteams von der Operation ablenkt.

Derartige HF-chirurgische Instrumente und -geräte umfassen nicht nur solche, die eine elektrische Leistung liefern, sondern auch solche, in denen "mechanische" Funktionen, wie Absaugen oder Spülen gleichzeitig durchgeführt werden.

Um die Einstellungsarbeit beim erstmaligen Verbinden eines HF-chirurgischen Instruments mit einem HF-chirurgischen Gerät zu vereinfachen, ist aus der DE 43 39 049 A1 ein Vorschlag bekannt, nach welchem im HF-chirurgischen Instrument eine Kodierungseinrichtung vorgesehen ist, welche über Anschlussstecker das HF-chirurgische Gerät programmiert, also alle Parameter des HF-chirurgischen Gerätes so einstellt, dass sie zum HF-chirurgischen Instrument passen. Hierbei wird allerdings vorausgesetzt, dass das HF-chirurgische Instrument auf das HF-chirurgische Gerät angepasst ist, sich sozusagen mit ihm "versteht". Will man HF-chirurgische Instrumente an HF-chirurgischen Geräten fremder Hersteller betreiben, so ist eine solche Kommunikation nicht möglich.

Der Erfindung liegt die Aufgabe zu Grunde, eine Möglichkeit aufzuzeigen, HF-chirurgische Instrumente verschiedener Bauarten und/oder Hersteller mit HF-chirurgischen Geräten verschiedener Bauarten und/oder Hersteller verwendbar zu machen.

Diese Aufgabe wird durch ein Verbindungskabel nach Anspruch 1 gelöst.

Insbesondere wird die Aufgabe durch ein Verbindungskabel gelöst, das eine erste Verbindungseinrichtung aufweist, die zum Verbinden mit einer Anschlussbuchse eines HF-chirurgischen Gerätes ausgebildet ist, die über ein Kabel mit einer zweiten Verbindungseinrichtung verbunden ist, die zum Verbinden mit einem HF-chirurgischen Instrument ausgebildet ist, wobei weiterhin ein programmierbarer Speicher vorgesehen ist, der mit Kontakten der ersten Verbindungseinrichtung verbunden und derart ausgebildet ist, dass mit der Anschlussbuchse verbundene Einstelleinrichtungen des HF-chirurgischen Gerätes zu einer Anpassung von Geräteeinstellungen auf das HF-chirurgische Instrument einem gespeicherten Datensatz entsprechend ansteuerbar sind.

Ein wesentlicher Punkt der Erfindung liegt also darin, dass die Kodiereinrichtungen zum einen programmierbar sind, zum anderen in einem Verbindungskabel, sozusagen einem "Adapterkabel" angebracht sind. Dadurch ist es möglich, im HF-chirurgischen Instrument vorhandene bzw. über dessen Stecker ausgegebene "Kodiersignale" in solche "Kodiersignale" umzuwandeln, die in einem dafür vorgesehenen HF-chirurgischen Gerät kompatibel sind, von diesem also verstanden werden.

Vorzugsweise sind Programmieranschlüsse des Speichers mit Kontakten der ersten oder der zweiten Verbindungseinrichtung zum Programmieren des Speichers verbunden. Es muss also der Speicher keine von außen in komplizierter Weise anzuzapfenden Programmiereinrichtungen aufweisen, es kann das Verbindungskabel vielmehr über diese in einfacher Weise nach außen geführten Programmieranschlüsse bzw. die Kontakte der Verbindungseinrichtungen programmiert werden.

Der Speicher ist vorzugsweise in der ersten oder in der zweiten Verbindungseinrichtung angeordnet, es wird also kein gesondertes Gehäuse benötigt.

Bevorzugte Ausführungsformen der Erfindung ergeben sich aus den Unteransprüchen.

Nachfolgend wird ein Ausführungsbeispiel der Erfindung anhand einer Abbildung näher erläutert.

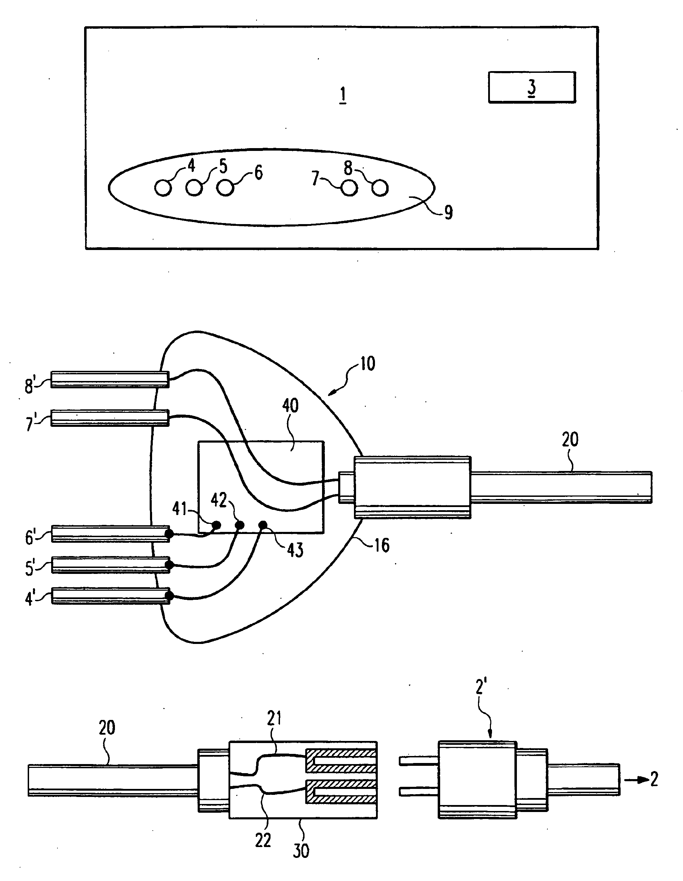

Die Abbildung zeigt im oberen Teil in schematisierter Darstellung ein HF-chirurgisches Gerät 1, das Einstelleinrichtungen 3 aufweist, die manuell betätigbar sind, um die oben erwähnten verschiedenen Parameter einzustellen. Weiterhin weist das HF-chirurgische Gerät 1 eine Anschlussbuchse 9 auf, die Kontaktelemente 4, 5, 6, 7 und 8 umfasst. Diese Kontaktelemente sind als Buchsen ausgebildet, in welchen als Stecker ausgebildete entsprechende Kontaktelemente 4', 5', 6', 7', 8' eines als erste Verbindungseinrichtung ausgebildeten Steckers 10 einsteckbar sind.

Im Gehäuse 16 des Steckers 10 ist ein programmierbarer Speicher 40 angeordnet, dessen Programmieranschlüsse 41, 42 und 43 mit den Kontaktelementen 4', 5' und 6' verbunden sind. Diese Programmieranschlüsse 41 - 43 sind gleichzeitig auch Signalausgänge, wobei die Programmierung in an sich bekannter Weise (über erhöhte Spannungen) erfolgt.

Mit dieser ersten Verbindungseinrichtung 10 ist über ein Kabel 20 eine zweite Verbindungseinrichtung 30 verbunden, die bei dem hier gezeigten Ausführungsbeispiel als Kupplung zum Einstecken eines Steckers 2' eines HF-chirurgischen Instruments 2 ausgebildet ist. Die Steckkontakte des Steckers 2' sind über elektrische Leitungen 21 und 22 mit den Steckkontakten 6' und 8' der ersten Verbindungseinrichtung 10 verbunden, so dass ein Hochfrequenzstrom aus dem HF-chirurgischen Gerät 1 über dessen Kontaktelemente 7 und 8 dem HF-chirurgischen Instrument 2 zuführbar ist.

Der programmierbare Speicher 40 wird nun derart programmiert, dass die Kodierung der Kontakte 4' - 6' bei Anschluss an die Anschlussbuchse 9 und dementsprechender Stromzuführung die Einstelleinrichtungen 3 bzw. die im HF-chirurgischen Gerät 1 vorgesehenen elektrischen Einrichtungen derart beeinflusst bzw. voreinstellt, dass diese zu dem angeschlossenen HF-chirurgischen Instrument 2 passen.

Wenn nun das HF-chirurgische Instrument 2 mit zwei verschiedenen HF-chirurgischen Geräten 1 betrieben werden soll, so werden zum einen die ersten Verbindungseinrichtungen 10 ihrer Form entsprechend an die verschiedenen Anschlussbuchsen 9 der verschiedenen HF-chirurgischen Geräte 1 angepasst. Darüber hinaus werden die programmierbaren Speicher 40 den von den jeweiligen HF-chirurgischen Geräten 1 geforderten "Kodier-Normen" entsprechend angepasst und zwar derart, dass das jeweils angeschlossene HF-chirurgische Instrument 2 (von derselben Bauart) in derselben Art und Weise bzw. mit denselben Parametern betrieben wird, unabhängig davon, welches der verschiedenen HF-chirurgischen Geräte benutzt wird. Mit der Erfindung soll also eine wechselweise Benutzbarkeit und beliebige Kombinationen von Instrumenten und Geräten ermöglicht werden.

Bei einer weiteren, hier nicht gezeigten Ausführungsform der Erfindung sind im programmierbaren Speicher 40 mehrere Kodierungsprogramme bzw. Sätze von Kodierungsdaten gespeichert. Je nachdem, welches HF-chirurgische Instrument 2 mit der zweiten Verbindungseinrichtung 30 verbunden wird, ändern sich die Kodierungsdaten, welche vom programmierbaren Speicher 40 über die Kontaktelemente 4', 5' und 6' bzw. 4, 5 und 6 dem HF-chirurgischen Gerät 1 mitgeteilt werden. Diese Ausführungsform wird insbesondere dann bevorzugt, wenn zu einem HF-chirurgischen Instrument mit einem bestimmten Stecker 2' verschiedene Ausführungsformen existieren, z.B. Ausführungsformen, die verschieden hohe Maximalspannungen zulassen und die selbst Kodierungseinrichtungen aufweisen, so dass aus dem programmierbaren Speicher 40 entsprechende Kodierungsdatensätze abrufbar sind.

### Bezugszeichenliste

- 1: HF-chirurgisches Gerät
- 2: HF-chirurgisches Instrument
- 3: Einstelleinrichtung
- 4, 4': Kontaktelement
- 5, 5': Kontaktelement
- 6, 6': Kontaktelement
- 7, 7': Kontaktelement
- 8, 8': Kontaktelement
- 9: Anschlussbuchse
- 10: erste Verbindungseinrichtung
- 16: Gehäuse
- 20: Kabel
- 21: Leitung
- 22: Leitung
- 30: zweite Verbindungseinrichtung
- 40: Speicher
- 41 - 43: Programmieranschluss

## Patentansprüche

1. Verbindungskabel mit einer ersten Verbindungseinrichtung (10), die zum Verbinden mit einer Anschlussbuchse (9) eines HF-chirurgischen Gerätes (1) ausgebildet ist,
umfassend
einen programmierbaren Speicher (40), der mit Kontakten (6', 7', 8') der ersten Verbindungseinrichtung (10) verbunden und derart ausgebildet ist, dass mit der Anschlussbuchse (9) verbundene Einstelleinrichtungen (3) des HF-chirurgischen Gerätes (1) zu einer Anpassung von Geräteeinstellungen auf das HF-chirurgische Instrument (2) einem gespeicherten Datensatz entsprechend ansteuerbar sind **dadurch gekennzeichnet daß** die erste Verbindungseinrichtung über das Verbindungskabel mit einer zweiten Verbindungseinrichtung (30) verbunden ist, welche zum Verbinden mit einem HF-chirurgischen Instrument (2) ausgebildet ist.

2. Verbindungskabel nach Anspruch 1,
**dadurch gekennzeichnet, dass**
die Programmieranschlüsse (41, 42, 43) des Speichers (40) mit Kontakten (4', 5', 6') der ersten Verbindungseinrichtung (10) oder der zweiten Verbindungseinrichtung (30) zum Programmieren des Speichers (40) verbunden sind.

3. Verbindungskabel nach einem der vorhergehenden Ansprüche,
**dadurch gekennzeichnet, dass**
der Speicher (40) in der ersten Verbindungseinrichtung (10) oder in der zweiten Verbindungseinrichtung (30) angeordnet ist.

## Claims

1. Connection cable with a first connecting means (10) designed for connection to a socket (9) of an HF-surgical device (1),
comprising
a programmable memory (40), which is connected to contacts (6', 7', 8') of the first connecting means (10) and is designed in such a way that setting means (3) of the HF-surgical device (1) that are connected to the socket (9) can be activated in a way corresponding to a stored data record to perform an adaptation of device settings to the HF-surgical instrument (2), **characterized in that** the first connecting means is connected by way of the connection cable to a second connecting means (30), which is designed for connecting to an HF-surgical instrument (2).

2. Connection cable according to Claim 1, **characterized in that** the programming connections (41, 42, 43) of the memory (40) are connected to contacts (4', 5', 6') of the first connecting means (10) or of the second connecting means (30) for programming the memory (40).

3. Connection cable according to one of the preceding claims, **characterized in that** the memory (40) is disposed in the first connecting means (10) or in the second connecting means (30).

## Revendications

1. Câble de connexion comprenant un premier dispositif de connexion (10) qui est réalisé pour la connexion à une douille de raccordement (9) d'un appareil chirurgical HF (1), comprenant
une mémoire programmable (40) qui est connectée à des contacts (6', 7', 8') du premier dispositif de connexion (10) et qui est réalisée de telle sorte que des dispositifs d'ajustement (3) de l'appareil chirurgical HF (1) connectés à la douille de raccordement (9) puissent être commandés en vue d'une adaptation d'ajustements d'appareil à l'instrument chirurgical HF (2) de manière correspondant à un ensemble de données mémorisées, **caractérisé en ce que** le premier dispositif de connexion est connecté par le biais du câble de connexion à un deuxième dispositif de connexion (30) qui est réalisé pour la connexion à un instrument chirurgical HF (2).

2. Câble de connexion selon la revendication 1,
**caractérisé en ce que**
les raccords de programmation (41, 42, 43) de la mémoire (40) sont connectés à des contacts (4', 5', 6') du premier dispositif de connexion (10) ou du deuxième dispositif de connexion (30) en vue de la programmation de la mémoire (40).

3. Câble de connexion selon l'une quelconque des revendications précédentes,
**caractérisé en ce que**
la mémoire (40) est disposée dans le premier dispositif de connexion (10) ou dans le deuxième dispositif de connexion (30).
